(19) Europäisches Patentamt
European Patent Office
Office européen des brevets

(11) Publication number: **0 295 719**
**A2**

(12) # EUROPEAN PATENT APPLICATION

(21) Application number: **88109795.0**

(51) Int. Cl.4: **G01N 33/564**

(22) Date of filing: **20.06.88**

The microorganism(s) has (have) been deposited with the American Type Culture Collection under number(s) 67732.

(30) Priority: **19.06.87 US 64802**

(43) Date of publication of application:
**21.12.88 Bulletin 88/51**

(84) Designated Contracting States:
**AT BE CH DE ES FR GB GR IT LI LU NL SE**

(71) Applicant: **THE AGOURON INSTITUTE**
**505 Coast Boulevard South**
**La Jolla California 92037(US)**

(72) Inventor: **Hoch, Sallie O.**
**1054 Havenhurst Drive**
**La Jolla Califorinia 92037(US)**
Inventor: **Rokeach, Luis A.**
**5484 55th Street Apartment E**
**San Diego California 92115(US)**
Inventor: **Haselby, Jeanne A.**
**26447 North Broadway**
**Escondido California 92026(US)**

(74) Representative: **Patentanwälte Grünecker, Kinkeldey, Stockmair & Partner**
**Maximilianstrasse 58**
**D-8000 München 22(DE)**

(54) **The SM-D antigen, the cloning of the SM-D antigen and the detection of systemic lupus erythematosus by using the SM-D antigen.**

(57) snRNP proteins are isolated, as by immunoaffinity chromatography, using antibodies from a serum sample of a living being affected by systemic lupus erythematosus. The Sm-D polypeptide is in turn isolated from the snRNP protein complex as by gel electrophoresis and electroelution. The amino terminus of the Sm-D polypeptide is then sequenced, and labelled DNA probes with nucleotide sequences complementary to that encoding the amino acid sequence are synthesized. A human cDNA library in a lambda cloning vector is transferred to appropriate filters such as nitrocellulose filters. These filters are screened as by hybridization with the labelled probes to identify the cDNA clones in the library with sequences matching those of the labelled probes. The cDNA encoding the Sm-D protein is then subcloned. The Sm-D polypeptide is then isolated as by immunoaffinity chromatography and, if further desired, as by HPLC chromatography. An assay is then made with the isolated Sm-D polypeptide by reacting the Sm-D polypeptide with a serum sample of a living being to determine whether the living being is affected by systemic lupus erythematosus. The assay may be performed as by an enzyme-linked immunoabsorbant such as anti-human IgG/IgM antibodies covalently attached to an enzyme indicator. Lactoperoxidase/alkaline phosphatase are each an example of an enzyme indicator to indicate, as by distinctive color, an affected person.

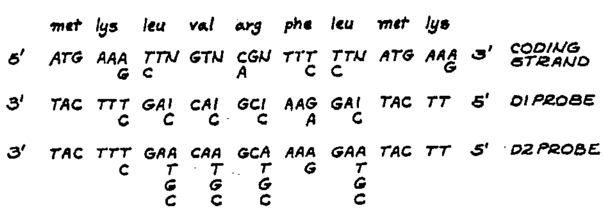


FIG. 1

EP 0 295 719 A2

# THE SM-D ANTIGEN, THE CLONING OF THE SM-D ANTIGEN AND THE DETECTION OF SYSTEMIC LUPUS ERYTHEMATOSUS BY USING THE SM-D ANTIGEN

This invention relates to a method of testing a serum sample of a living being to detect whether or not the living being has systemic lupus erythematosus. More particularly, the invention relates to a method of reacting a serum sample of a living being with the Sm-D polypeptide to detect whether or not the living being has systemic lupus erythematosus. The invention further relates to a method of isolating and cloning the cDNA for the Sm-D polypeptide. The method also relates to a combination of the Sm-D antigen and materials for indicating the reaction of the Sm-D antigen with antibodies of a person affected by systemic lupus erythematosus.

In an autoimmune disease, the body's immune system generates antibodies which react with the body's own cellular components. The resulting formation of immune complexes can lead to a variety of pathological conditions. Some rheumatic diseases are autoimmune disceses. One type of such rheumatic disease is systemic lupus erythematosus.

Antinuclear antibodies are a hallmark of autoimmune diseases. The antibodies are antinuclear because they act against components in the nucleus of the cells in the living beings. Assays based upon the measurement of these antibodies are well established in the clinical laboratory for diagnosis of a variety of rheumatic diseases. (1,2). The assays in general do not employ a purified antigen as the target of these antibodies. Thus, the assays are relatively crude. This results from the fact that cellular extracts routinely serve as the source of the antigen so that there is no defined standard to serve as an internal control. The problem has been complicated because the molecular nature of the target antigens for these autoimmune antibodies is relatively complex.

In recent years, persons skilled in the art have begun to decipher the molecular nature of the target antigens for the autoimmune antibodies and, in particular, to recognize their complexity relative to the large structural elements of which they may be a part. The large structural element may be specifically ribonucleoprotein (RNP) or deoxyribonucleoprotein (DNP) particles. Persons skilled in the art have also begun to recognize the complexity of these antigens relative to their roles in the functioning of the cells in living beings. (3). With the advent of such information, the potential exists for the development of defined and abundant antigen reagents and for the use of these reagents in the development of sensitive and quantitative clinical assays.

Attempts have been made to isolate relatively simple polypeptides or proteins as the antigens in detecting the antibodies in autoimmune diseases such as rheumatic diseases. Although these polypeptides or proteins are simple antigens, they are actually part of yet more complex cellular structures. As a result, it has been difficult to isolate the polypeptides or proteins which react with such antibodies. It has also been difficult to develop an assay in which such antigens are used to detect the antibodies of the autoimmune diseases such as rheumatic diseases.

It has been particularly difficult to develop a defined and abundant antigen for the rheumatic disease designated as systemic lupus erythematosus since the antigen (designated Sm) for systemic lupus erythematosus is associated with a conserved class of proteins called the snRNP (small nuclear ribonucleoproteins (4). The Sm small nuclear ribonucleoproteins (snRNP) associated with the Sm antigens contain five species of U snRNA (U1, U2, U4, U5 and U6) and at least eleven polypeptides. The U snRNA are rich in uridine.

Applicants and other persons skilled in the art have identified Sm snRNP. (5-8). Applicants have now isolated the Sm-D polypeptide antigen associated with systemic lupus erythematosus. The Sm-D polypeptide has a molecular weight of approximately thirteen thousand (13,000). Applicants have also formulated a simple and effective colorimetric assay in which such antigen can be used with a serum sample of a living being to identify whether the living being is affected by systemic lupus erythematosus.

In one embodiment of the invention, snRNP proteins are isolated, as by immunoaffinity chromatography, using antibodies from a serum sample of a living being affected by systemic lupus erythematosus. The Sm-D polypeptide is in turn isolated from the snRNP protein as by gel electrophoresis and electroelution. The amino terminus of the Sm-D polypeptide is then sequenced, and labelled DNA probes with nucleotide sequences complementary to that encoding this amino acid sequence are synthesized. A human cDNA library in a lambda cloning vector is transferred to appropriate filters such as nitrocellulose filters. These filters are screened as by hybridization with the labelled probes to identify the cDNA clones in the library with sequences matching those of the labelled probes.

The cDNA encoding the Sm-D protein is then subcloned. The Sm-D polypeptide is then isolated as by immunoaffinity chromatography and, if further desired, as by HPLC chromatography. An assay is then made with the isolated Sm-D polypeptide by reacting the Sm-D polypeptide with a serum sample of a living being to determine whether the living being is affected by systemic lupus erythematosus. The assay may be performed as by an enzyme-linked immunoabsorbant assay such as one using anti-human IgG/IgM antibodies covalently attached to an enzyme indicator. Lactoperoxidase/alkaline phosphatase are each an example of an enzyme indicator to indicate, as by a distinctive color, an affected person.

In the drawings:

The single Figure indicates the sequence of a number of amino acids at the amino terminus of the Sm-D polypeptide and further indicates probes synthesized to hybridize with the cDNA encoding such amino acid sequence.

## Isolation of Sm SnRNP

In one embodiment of the invention, the Sm snRNP was isolated by immunoaffinity chromatography. As a first step in such process, a plasmapheresis of a patient affected by systemic lupus erythematosus was obtained as the source of human anti-Sm antibodies. The immunoglobulin G (IgG) was purified by half-saturation ammonium sulfate fractionation and passage over a DEAE-Sephacel column. The isolated IgG was coupled to Sepharose CL-4B by a carbonyl - diimidazole procedure. (10).

Extracts were prepared from suspension cultures of a human cell line designated as HeLa as by sonication of total cells in 0.35M NaCl, 10mM Tris-HCl, pH 7.4, 1.5mM $MgCl_2$, 0.2mM phenylmethylsulfonyl fluoride. (8). The cell extracts were passed immediately after preparation through the anti-Sm immunoaffinity column which was prepared as discussed in the preceding paragraph. The columns were then washed with an extraction buffer and were eluted in 10mM Tris HCl, pH 7.4, containing 0.1 NaCl, 6M urea and 0.2 mM phenylmethylsulfonyl fluoride. Carrier RNA (20 µg/ml) was added to the eluate to facilitate the subsequent precipitation of the Sm snRNP at dilute protein concentrations. The Sm snRNP was then precipitated overnight at -20° C. by the addition of two (2) volumes of ethanol.

The immunoaffinity column eluates were analyzed for total protein content (including the characteristic Sm snRNP profile) by Coomassie blue stain after sodium dodecyl sulfate (SDS) gel electrophoresis. (11). Antigenic polypeptides were detected by immunoblot after transfer to nitrocellulose. (12, 13). In this way, the Sm antigens were purified on a bulk basis in sufficient purity for direct biochemical analysis. This procedure minimized protease and nuclease exposure of the Sm snRNP. (9).

## Isolation of Individual Sm snRNP Polypeptides

Although applicants were able to isolate the Sm snRNP particle readily by chromatographic techniques, they were not able to fractionate clearly the Sm snRNP particle by chromatographic techniques. Instead, applicants fractionated the Sm snRNP particle by using SDS-polyacrylamide gel electrophoresis and electroelution. The protocol (and apparatus) used by applicants to accomplish this was that used by Hunkapiller et al (14) to isolate microgram quantities of protein. This protocol was ideal for applicants' purposes because, for each polypeptide band, they were able to elute a small amount of protein (10-20 µg) from a large block of acrylamide gel (10-11cm in width/1.5mm in thickness) into a small volume (300-500 µl).

The only modification made by applicants in the Hunkapillar et al protocol was to change the staining conditions prior to excising individual bands across the gel. This change was made because a number of the peptides of interest were quite closely spaced even on long (20 cm) gels. In order to be able to clearly distinguish between such peptides of interest after the usual destaining period of two (2) hours, the Coomassie blue was decreased to five hundredths of one percent (0.05%) from the original protocol of Hunkapillar et al. After electroelution of individual bands of peptides, each band was checked for homogeneity by silver stain after the SDS gel electrophoresis (11) and for immunoreactivity by protein blot. (12, 13).

Protein Sequencing

Applicants chose a gas-phase system to sequence the amino acids in the Sm-D polypeptide because only pmole amounts of protein were required. (15, 16). The sequencing was performed at the Protein Chemistry Laboratory, Washington University, St. Louis, Missouri (a fee-for-service facility). Applicants obtained a sequence of eleven (11) residues for the Sm-D polypeptide at the amino terminus of the Sm-D polypeptide. The sequence was as follows:

met lys leu val arg phe leu met lys leu ser

## Molecular Cloning of the Sm-D protein cDNA.

(a) Design of probes

Applicants synthesized two (2) twenty six (26) - mer oligonucleotide probes (ABI Synthesizer, The Agouron Institute) in order to clone the cDNA encoding the Sm-D protein. These probes were synthesized with a base composition deduced from the amino acid sequence available from the sequencing of the Sm-D polypeptide. These probes were designated as the D1 and D2 probes. The probes were labelled as by radioactive phosphorus.

The D1 and D2 probes were formed as follows:

| | met | lys | leu | val | arg | phe | leu | met | lys | | |
|---|---|---|---|---|---|---|---|---|---|---|---|
| 5' | ATG | AAA | TTN | GTN | CGN | TTT | TTN | ATG | AAA | 3' | Coding Strand |
| | | G | C | | A | C | C | | G | | |
| 3' | TAC | TTT | GAI | CAI | GCI | AAG | GAI | TAC | TT | 5' | D1 probe |
| | | C | C | C | C | A | C | | | | |
| 3' | TAC | TTT | GAA | CAA | GCA | AAA | GAA | TAC | TT | 5' | D2 probe |
| | | C | T | T | T | G | T | | | | |
| | | | G | G | G | | G | | | | |
| | | | C | C | C | | C | | | | |

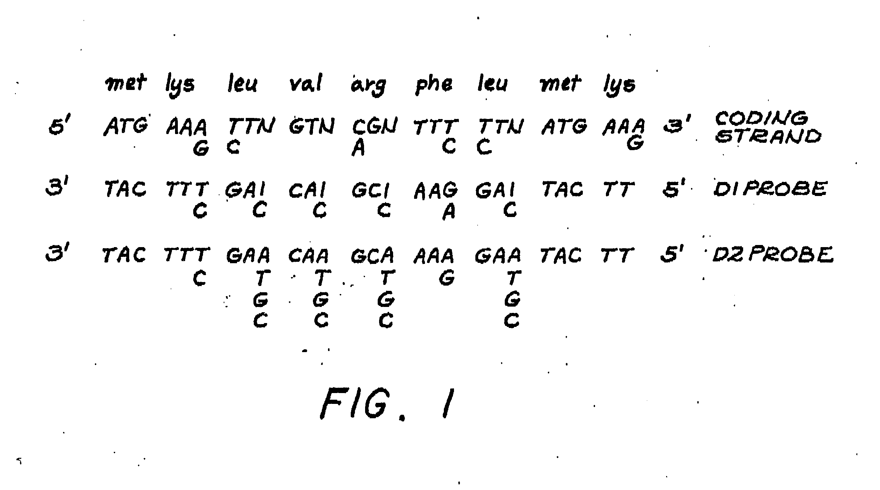

This is also shown in Figure 1. In the above sequences, "N" represents any of the four (4) nucleotides A, T, G and C. "I" substitutes for all of the four (4) nucleotides except the nucleotide "C". This corresponds to deoxyinosine.

As will be seen, the D1 probe was designed to use deoxyinosine at the third position of the most ambiguous codons. (17). The D1 probe was accordingly formed as a mixture of sixty four (64) different oligonucleotides. In the D2 probe, all four (4) nucleotides were included at the third (3rd) position of redundant codons. Furthermore, based upon codon usage (18), applicants considered only cytosine at the first position of leucine triplets. As a result, the total number of molecular species for the D2 probe was reduced to ten hundred and twenty four (1,024).

(b) Library Screening

As the source of cDNA, a library containing λgt10 cloning vectors and made from human B lymphocyte polyA-RNA was purchased from Clonetech Laboratories, Inc., of Palo Alto, California. Approximately two hundred and fifty thousand (250,000) independent λ recombinant plaques were plated on E. coli Y1090 bacterial lawn and transferred to nitrocellulose filters. (19). These filters were screened by hybridizing them with the [$^{32}$p]-labelled D1 probe. From the first round of screening, sixty (60) plaques were identified as having become hybridized with the [$^{32}$p]-labelled D1 probe.

The nitrocellulose filters were then stripped from the D1 probe. The plaques were then rescreened with

the [$^{32}$p]-labelled D2 probe. In this screening, twenty three (23) placques were identified as having become hybridized with the [$^{32}$p]-labelled D2 probe. Of these twenty three (23) positive probes, fifteen (15) had previously been identified as positive lambda in having become hybridized with the [$^{32}$p]-labelled D1 probe. The twenty three (23) positive λ recombinants were subjected to two (2) additional cycles of purification involving the use of the D2 probe. After such two (2) additional cycles of hybridization, a total of eighteen (18) pure positive clones were obtained.

(c) Subcloning and Sequencing

DNA from the eighteen (18) lambda positive clones was prepared (19) and digested with EcoRI enzyme. The size of the cDNA inserts was determined after such digestion. The inserts were classified into seven (7) groups each containing inserts within a particular range of nucleotide lengths. These groups ranged in lengths from 0.65 Kb to 2.5 Kb. One (1) representative from each of the seven (7) groups was subcloned into a generic vector designated as M13mp18 (20) and the nucleotide sequence was determined by employing the dideoxy method. (21). A clone identified by applicants as λ D45-2 was found to contain a DNA sequence perfectly matching the codon composition for the known sequence of eleven (11) amino acids in the Sm-D polypeptide. The EcoRI fragment from the λ D45-2 clone was further inserted into a generic vector designated as pUC18 (22) to test the antigenicity of the recombinant D protein produced in Escherichia coil (E. coli).

(d) Expression of the Recombinant Sm-D Protein

Expression of the Sm-D protein may be provided in E. coli. There are two (2) major reasons for this choice: (1) This bacterium is the most studied and understood organism in terms of its physiology, genetics and molecular biology; (2) a wide variety of convenient expression vectors already exist. (23-26). These vectors utilize strong bacterial transcriptional and translational signals to achieve high levels of synthesis of the cloned gene product.

To prevent problems of inhibition of bacterial growth by the cloned gene product, expression of the cloned gene product may be conditionally activated by using inducible promotors of transcription. For example, plasmids pIN-III (23) and pKK233-2 (24) respectively contain the lac and tac promoters. Both are inducible by the addition of isopropyl- $\beta$ -D-thiogalactoside (IPTG) to the cultures. Plasmmid pAS1 (25) bears the λpL promoter which is activated by shifting the incubation temperature from 30°C. to 42°C. Another system (26) is based on the use of two (2) plasmids. One plasmid pGP1-2 encodes the T7 RNA polymerase under the control of the $\lambda_{pL}$ promoter. The gene of interest is cloned in a second plasmid pT7-3 under the control of the $\phi$ 10 T7 RNA polymerase promoter.

As a first step, the cDNA encoding the Sm-D protein may be cloned in all of the vectors cited above. Secondly, expression of the recombinant Sm-D protein may be optimized by determining the best incubation conditions before and after the induction of the bacterial transcriptional promoter. Levels of expression of the cloned Sm-D polypeptide can be monitored by labelling the newly synthesized polypeptides with [$^{35}$S] - methionine and taking at different times samples which are then subjected to SDS - polyacrylamide electrophoresis and autoradiography.

Assays for anti-Sm antibodies

A direct assay may be used to determine the reactivity of the antibodies in a living being such as a patient. A major limitation to a direct assay is generally that a relatively large quantity of antigen is required to make such an assay. This is particularly true when it is desired to have the antigen at or near complete homogeneity.

Large quantities of antigen may be produced as discussed two (2) paragraphs previously. For example, the Sm-D antigen may be isolated as by immunoaffinity chrogratography discussed in the section entitled "Isolation of Sm snRNP". In such immunoaffinity chromatography, a polyclonal human serum may be used. Alternatively, a mouse monoclonal antibody may be employed which has specificity for the Sm-D band (28). If this is considered desirable, preparative HPLC chromatography may be employed on the antigen as a final purification step.

An enzyme-linked immunoabsorbent assay (27) is the preferred assay. The purified Sm-D polypeptide is preferably absorbed on the surface of microtiter plates. Any exposed surfaces on the microtiter plates are preferably blocked with a concentrated non-reactive protein solution (e.g. bovine serum albumin) (28). The serum sample to be tested is preferably allowed to react directly with the Sm-D antigen.

Any antigen-antibody immune complex formed as a result of the reaction of the serum sample with the Sm-D antigen is preferably detected as by anti-human IgG/IgM antibodies covalently attached to an enzyme indicator (e.g. lactoperoxidase/alkaline phosphatase). The quantitation of the attached enzyme indicator may be indicated as by a colorimetric assay. Furthermore, the microtiter plates can be read on an automatic plate reader. Background measurements can also be obtained through appropriate controls by using a serum from an unaffected person to provide a negative response and by using a known human anti-Sm autoantibody (29) or the mouse anti-Sm-D monoclonal antibody to obtain a positive response.

The methods described above have certain important advantages. They provide for the cloning of the Sm-D antigen to detect systemic lupus erythematosus. They also provide for the reaction of this antigen with a serum sample of a person to detect whether the person is affected by systemic lupus erythematosus. Another antibody with an enzyme can be included to produce a distinctive color when the antibody of systemic lupus erythematosus reacts with the Sm-D antigen to produce an antigen-antibody complex, and the other enzyme-linked antibody reacts with the antigen-antibody complex and is detected by a colorimetric assay for activity of the enzyme. This distinctive color can be detected to indicate that the person being tested is affected by systemic lupus erythematosus.

The methods described above also have other important advantages. Since the antigen is only the Sm-D polypeptide rather than a complex containing the Sm-D polypeptide and a number of other polypeptides and proteins, the reaction of the Sm-D polypeptide with the antibodies of systemic lupus erythematosus is more specific and sensitive than the reaction of such complexes with such antibodies as in the prior art. This causes applicants' method of detection to be more definite and certain than the tests on the complexes of the prior art.

Although this invention has been disclosed and illustrated with reference to particular embodiments, the principles involved are susceptible for use in numerous other embodiments which will be apparent to persons skilled in the art. The invention is, therefore, to be limited only as indicated by the scope of the appended claims.

## LITERATURE CITED


1. Fritzler, M.J. 1986. Methods Achiev. Exp. Pathol. 12:224.
2. McCarty, G.A. 1986. Med. Clin. North Am. 70:237.
3. Brunel, C., J. Sri-Widada and P. Jeanteur. 1985. Prog. Mol. Subcell. Biol. 9:1.
4. Lerner, M.R. and J.A. Steitz. 1979. Proc. Natl. Acad. Sci. U.S.A. 76:5495.
5. Hinterberger, M., I. Pettersson, and J.A. Steitz. 1983. J. Biol. Chem. 258:2604.
6. Kinlaw, C.S., B.L. Robberson, and S.M. Berget. 1983. J. Biol. Chem. 258:7181.
7. Bringmann, P., J. Rinke, B. Appel, R. Reuter, and R. Luhrmann. 1983. EMBO J. 2:1129.
8. Billings, P.B. and S.O. Hoch. 1980. J. Biol. Chem. 259:12850.
9. Billings, P.B. and S.O. Hoch. 1983. J. Immunol. 131:347-351.
10. Hearn, M.T.W., E. L. Harris, G.S. Bethell, W.S. Hancock and J.A. Ayers. 1981. J. Chromatogr. 218:509.
11. Laemmli, U.K. 1970. Nature (Lond.) 227:680.
12. Billings, P.B., J.R. Barton and S.O. Hoch. 1985. J. Immunol. 135:428.
13. Towbin, H., T. Staehelin and J. Gordon. 1979. Proc. Natl. Acad. Sci. U.S.A. 78:4350.
14. Hunkapiller, M.W., E. Lujan, F. Ostrander and L.E. Hood. 1983. Methods Enzymol. 91:277.
15. Hewick, R.M., M.W. Hunkapiller, L.E. Hood and W.J. Dreyer. 1981. J. Biol. Chem. 256:7990.
16. Hunkapiller, M.W., R.M. Hewick, W.J. Dryer and L.E. Hood. 1983. Methods Enzymol. 91:399.
17. Ohtsuka, E., S. Matsuki, M. Ikebasa, Y. Takahashi and K. Matsubana. 1985. J. Biol. Chem. 260:2605.
18. Lathe, R. 1985. J. Mol. Biol. 183:1.
19. Maniatis, T., E.F. Fritsch and J. Sanbrook, 1982. Molecular Cloning: A Laboratory Manual. Cold Spring Harbor Laboratory Press, Cold Spring Harbor, NY.
20. Norrander, J., T. Kempe, and J. Messing. 1983. Gene. 26:101.
21. Sanger, F., S. Nickler and A.R. Coulson. 1977. Proc. Natl. Acad. Sci. U.S.A. 74:5463.
22. Yanish-Perron, C., J. Vierra, and J. Messing. 1985. Gene. 33:103.

23. Masui, Y., J. Coleman, and M. Inouye. 1983. In Experimental Manipulation of Gene Expression. Academic Press, Inc. New York, NY pp.15.

24. Amann, E., and J. Brosius. 1987. Gene. In press.

25. Shatzman, A., Y-S Ho, and M. Rosenberg. 1983. In Experimental Manipulation of Gene Expression. Academic Press, Inc. New York, NY. pp.1.

26. Tabor, S. and C.C. Richardson. 1985. Proc. Natl. Acad. Sci. U.S.A. 82:1074.

27. Engvall, E. and P. Perlmann. 1971. Immunochemistry 8:871.

28. Billings, P.B., R.W. Allen, F.C. Jensen and S.O. Hoch. 1982. J. Immunol. 128:1176.

29. Tan, E.M., M.J. Fritzler, J.S. McDougal, F.C. McDuffie, R.M. Nakamura, M. Reichlin, C.B. Reimer, G.C. Sharp, P.H. Schur, M.R. Wilson and R.J. Winchester. 1982. Arthritis Rheum. 25:1003.

## Claims

1. A method of detecting whether a living being is affected by systemic lupus erythematosus, including the steps of

reacting the Sm-D polypeptide with a serum sample of the living being wherein the serum sample contains antibodies, and

determining from the reactivity of the Sm-D polypeptide with the antibodies in the serum sample of the living being whether the living being has systemic lupus erythematosus.

2. A method as set forth in claim 1 wherein

the Sm-D polypeptide has a molecular weight of approximately thirteen thousand (13,000).

3. A method as set forth in claim 2 wherein

the reactivity of the Sm-D polypeptide with the antibodies in the serum sample of the living being is detected by reacting the mixture with an anti-IgG/IgM antibody covalently attached to an enzyme indicator.

4. A method as set forth in claim 1 wherein

the Sm-D polypeptide has an amino acid sequence of met lys leu val arg phe leu met lys leu ser at its amino terminus.

5. A method of detecting whether a living body is affected by systemic lupus erythematosus, including the following steps:

using antibodies from a serum sample of a living being affected with sytemic lupus erythematosus to isolate the snRNP proteins,

isolating from the snRNP proteins the polypeptide containing a molecular weight of approximately thirteen thousand (13,000),

cloning the isolated polypeptide containing the molecular weight of approximately thirteen thousand (13,000),

reacting the cloned polypeptide with a serum sample of the living being, and

determining the presence of systemic lupus erythematosus in the living being from the reactivity of the cloned polypeptide with the serum sample of the living being.

6. A method as set forth in claim 5 wherein

the reactivity of the cloned polypeptide with the serum sample is determined by adding an anti-IgG/IgM antibody covalently attached to an enzyme indicator.

7. A method as set forth in claim 5 wherein

the isolated polypeptide is the Sm-D polypeptide.

8. A method as set forth in claim 5 wherein

the isolated polypeptide has at its amino terminus the sequence met lys leu val arg phe leu met lys leu ser.

9. In a method of detecting whether a living being is affected by systemic lupus erythematosus, the steps of:

cloning the Sm-D polypeptide, and

preparing the cloned Sm-D polypeptide for reactivity with a serum sample of the living being.

10. In a method as set forth in claim 9, the steps of:

isolating the Sm snRNP proteins,

isolating the Sm-D polypeptide from the snRNP proteins, and

cloning a DNA sequence encoding the Sm-D polypeptide based upon an amino acid sequence from the isolated Sm-D polypeptide.

11. In a method as set forth in claim 9,

the Sm-D polypeptide having a molecular weight of approximately thirteen thousand (13,000).

12. In a method as set forth in claim 9,
the Sm-D polypeptide having at its amino terminus an amino acid sequence of met lys leu val arg phe leu met lys leu ser.

13. A method of testing whether a living being is affected by systemic lupus erythematosus, including the steps of:
cloning the cDNA encoding the Sm-D polypeptide,
isolating the Sm-D polypeptide from the cDNA,
absorbing the isolated Sm-D polypeptide on the surface of a microtiter plate,
blocking any exposed surfaces,
reacting a serum sample of the living being with the blocked Sm-D polypeptide on the microtiter plate, and
detecting the formation of any antigen-antibody immune complex from such reaction.

14. A method as set forth in claim 13 wherein
the formation of the antigen-antibody immune complex is detected as by an anti-human IgG/IgM antibody covalently attached to an enzyme indicator.

15. A method as set forth in claim 13 wherein
the exposed surface of the Sm-D polypeptide on the microtiter plate is blocked with a concentrated nonreactive protein solution.

16. A method as set forth in claim 14
wherein the Sm-D polypeptide is isolated from the cDNA by immunoaffinity chromatography and
the exposed surface of the Sm-D polypeptide on the microtiter plate is blocked with a concentrated non-reactive protein solution.

17. A method as set forth in claim 16 wherein
the enzyme indicator is lactoperoxidase/alkaline phosphatase to provide the assay by a distinctive color for the antigen-antibody immune complex.

18. A test-kit for detecting whether a living being is affected by systemic lupus erythematosus, comprising
a Sm-D antigen for reacting with the antibodies of the living being when the living is affected by systemic lupus erythmatosus,
an additional antibody for reacting with the antigen-antibody immune complex when the Sm-D antigen is mixed with a serum sample of the living person and the living person is affected by systemic lupus erythematosus, and
an enzyme linked to the additional antibody to produce a distinctive color, upon the addition of appropriate substrates, when the antigen-antibody immune complex is formed and the additional antibody becomes coupled to the antigen-antibody immune complex.

19. A test-kit as set forth in claim 18, wherein
the additional antibody is an anti-human IgG/IgM antibody and
the enzyme is covalently attached to the additional antibody.

20. A test-kit as set forth in claim 9, wherein
the enzyme constitutes lactoperoxidase or alkaline phosphatase.

| | met | lys | leu | val | arg | phe | leu | met | lys | | |
|---|---|---|---|---|---|---|---|---|---|---|---|
| 5' | ATG | AAA | TTN | GTN | CGN | TTT | TTN | ATG | AAA | 3' | CODING STRAND |
| | | G | C | | A | C | C | | G | | |
| 3' | TAC | TTT | GAI | CAI | GCI | AAG | GAI | TAC | TT | 5' | D1 PROBE |
| | | C | C | C | C | A | C | | | | |
| 3' | TAC | TTT | GAA | CAA | GCA | AAA | GAA | TAC | TT | 5' | D2 PROBE |
| | | C | T | T | T | G | T | | | | |
| | | | G | G | G | | G | | | | |
| | | | C | C | C | | C | | | | |

FIG. 1